# EUROPEAN PATENT SPECIFICATION

(11) **EP 0 406 319 B1**
(45) Date of publication and mention of the grant of the patent: **08.02.1995**
(21) Application number: 89904679.1
(22) Date of filing: 20.03.1989
(51) Int. Cl.: C12P 19/30, C12N 9/16, C12N 9/22, C12N 9/96

(54) **5'-PHOSPHODIESTERASE ENZYME PREPARATION AND METHOD FOR ITS PRODUCTION**
5'-PHOSPHODIESTERASE-ENZYMZUBEREITUNG UND VERFAHREN ZUR HERSTELLUNG
PREPARATION DE 5'-PHOSPHODIESTERASE ET SON PROCEDE DE PRODUCTION

(30) Priority: 25.03.1988 US 172824
(43) Date of publication of application: 09.01.1991
(73) Proprietor: ENZYME BIO-SYSTEMS LTD., Englewood Cliffs, N.J. 07632 (US)
(72) Inventor: BOWLES, Linda, K., Chicago, IL 60605 (US)
(74) Representative: Lederer, Franz, Dr.
(86) International application number: US8901154
(87) International publication number: WO8909276

(56) References cited:
- US-A- 3 304 238
- US-A- 3 516 907
- CHEMICAL ABSTRACTS, vol. 94, Columbus, OH (US); Y.S. KYONG, p. 274, no. 79083b#
- CHEMICAL ABSTRACTS, vol. 101, 1981, Columbus, OH (US); p. 299, no. 12561h#

## Description

The present invention relates to a storage-stable 5'-phosphodiesterase enzyme preparation and to a method for its commercial production by extraction of rapidly proliferating parts of germinating seeds.

Ribonucleic acid (hereafter RNA) is a complex polymer occurring in living organisms. Certain enzymes are capable of splitting the RNA molecule into smaller subunits known as nucleotides. The 5'-nucleotides are of particular interest because of their ability to improve the richness and intensity of flavors without themselves contributing to taste of a food product. For this reason, there is considerable commercial interest in the preparation of the enzyme which hydrolyzes RNA to form 5'-nucleotides. This enzyme is known as 5'-phosphodiesterase.

It is well known that rapidly proliferating parts of germinating seeds, such as the rootlets and stems, contain 5'-phosphodiesterase. However, these materials contain other enzymes which are extracted along with the 5'-phosphodiesterase. Some of these other enzymes in the crude extract cause further hydrolysis of 5'-nucleotides. This makes the crude extracts unsuitable for use in preparing 5'-nucleotides from RNA.

In U.S. Patent 3,304,238, there is disclosed a method for preparing an aqueous enzyme medium capable of forming 5'-ribonucleotides from RNA. According to this disclosure, rapidly proliferating, substantially noncomminuted seed parts are heated in water to from about 70° to 85°C for several minutes. Malted barley rootlets are included in the list of seed parts used. The crude mixture of solid and liquid is used to hydrolyze RNA.

In U.S. Patent 3,459,637, ground rootlets or sprouts are extracted with water and the filtered extracts are used to hydrolyze RNA. In order to obtain 5'-nucleotides from RNA using this crude extract, it is necessary to carry out the reaction with RNA at a high pH, between 8.5 and 9.5. Zinc ions are added to accelerate the reaction.

U.S. Patent 3,516,907 discloses a method for obtaining 5'-nucleotides by reacting nucleic acids with a 5'-phosphodiesterase active extract. This extract is obtained by extracting plant material, such as barley sprouts, with water. The crude extract could be further purified by gel fractionation or selective precipitation with a solvent. Heat treatment of the vegetable extract, preferably in the presence of a heavy metal salt, is also disclosed.

A method for producing a yeast extract of improved flavor is disclosed in U.S. Patent 4,303,680. The method involves treating yeast RNA with a crude 5'-phosphodiesterase enzyme solution. The enzyme solution is obtained by extracting broken malt roots with water and then heating the clear filtrate at a temperature of 60 to 65°C for 5 to 10 minutes.

Although the foregoing references disclose a general method for extracting an enzyme solution having phosphodiesterase activity from plant parts, the general methods are only suitable for use on a laboratory scale. Furthermore, these crude extracts tend to lose activity on storage unless they are kept in a frozen state.

We have now discovered a process for the extraction of 5'-phosphodiesterase which is adaptable to large-scale commercial production. This process is capable of producing both liquid and solid enzyme preparations which have excellent storage stability.

Briefly, in accordance with this invention, there is provided a process for extracting an enzyme preparation from barley malt sprouts which comprises
mixing ground barley malt sprouts with water to give a first aqueous slurry of ground barley malt sprouts;
separating the larger particles of ground barley malt sprouts from said first aqueous slurry in a first separation step to give a second aqueous slurry containing finely-divided barley malt sprouts;
then separating the finely-divided barley malt sprouts from said second aqueous slurry in a second separation step to give a clarified aqueous enzyme extract having less than about 1% by weight of water-insoluble solids;
concentrating the clarified aqueous enzyme extract by ultrafiltration to give a concentrated enzyme extract; and
heating the concentrated enzyme extract at a pH between about 4.3 and about 5.4 at a temperature of between about 60°C and about 65°C for from about 15 to about 60 minutes to give an enzyme preparation having a ratio of 5'-phosphodiesterase units to 5'-nucleotidase units of at least 7:1.

Additionally, in accordance with this invention, there is provided an enzyme preparation extracted from barley malt sprouts which comprises 5'-phosphodiesterase and 5'-nucleotidase wherein the ratio of 5'-phosphodiesterase units to 5'-nucleotidase units is at least 7:1.

Since barley is commonly germinated in large quantities for the production of malt, barley malt sprouts are generally available as a by-product of the malting process. These sprouts are a preferred raw material for the present process.

More 5'-phosphodiesterase can be extracted from the plant material if the material is first broken by a grinding operation. This can be accomplished by any conventional means for reducing the size of particles, such a hammer mill or other conventional mill. In the practice of this invention, it is convenient to provide ground particles of such a size that they will pass through a screen having 1.65 mm openings.

In the process of this invention, the ground plant material is mixed with water to extract the water-soluble enzyme. For convenience in mixing, it is preferred to use at least about 8 parts by weight of water per part of plant material. Under preferred conditions, the pH of the mixture is between about 4.0 and about 5.0, the temperature of the mixture is maintained between about 12°C and about 20°C. Although the time of extraction is not critical, sufficient time should be given for good contact between the water and the solid material.

When using the enzyme preparation as aqueous extract a preservative is added to the water used for the enzyme extraction. Commonly-used food preservatives, such as sodium benzoate and methyl p-hydroxybenzoate are suitable for this process. A combination of these two preservatives is particularly effective.

The enzyme extract is separated from the slurry of ground plant material in water by a two-step process. In the first separation step, the larger particles of plant material are separated from the slurry. This step can be carried out by means of various separation devices, such as a belt press, a rotary drum concentrator, or a centrifugal paddle screen. A particularly suitable apparatus for this purpose is the centrifugal paddle screen, ICM Model No. 77, obtained from the Indiana Canning Machine Company, Indianapolis, Indiana, or equivalent, preferably having a screen opening of at least about 50 microns (»m). The filtrate from this first separation step is an aqueous slurry which still contains finely-divided ground plant material in suspension.

The slurry containing the finely-divided material is clarified in a second separation step. Any filtration means which provides rapid separation of finely-divided solids from a liquid may be used. A rotary precoat filter, i.e., a vacuum filter coated with a filter aid is particularly suitable for use in this step.

The clarified aqueous enzyme extract obtained from the second separation step is next subjected to concentration by means of ultrafiltration and to heat treatment. Although the order in which the ultrafiltration and heat treatment steps are carried out is not critical, it is more convenient and more economical to concentrate the solution by ultrafiltration before the heat treatment step. Ultrafiltration is carried out by multiple passes of the material through an ultrafiltration apparatus having a membrane which retains material with a molecular weight greater than about 5000 daltons. The ultrafiltration process is carried out until the desired enzyme concentration is obtained. A product with an enzyme concentration of between about 10 times and about 40 times that of the clarified aqueous enzyme extract is usually satisfactory.

The concentrated enzyme extract is then heated at a temperature of between about 60°C and about 65°C for about 15 to 60 minutes. The pH of the solution is adjusted to between about 4.3 and about 5.4 before the heat treatment. If a preservative has not been added earlier in the process, it may be added to the concentrated solution before the heat treatment step.

Heating of the concentrated enzyme extract may be carried out as a continuous process. This is accomplished conveniently by passing the extract through a continuous heat exchanger with a rate of flow being adjusted to give the desired heating time. The heated extract is then cooled rapidly to a temperature below about 25°C to terminate the heating step.

The 5'-phosphodiesterase obtained by the foregoing process has a ratio of 5'-phosphodiesterase units to 5'-nucleotidase units of at least 7:1, and preferably of at least 15:1.

It is often desirable to have a solid 5'-phosphodiesterase enzyme preparation. This can be prepared by removing the water from the concentrated enzyme extract under reduced pressure. This is conveniently carried out by a freeze-drying operation using a lyophilizer. The solid enzyme preparation obtained by this process has a concentration of 5'-phosphodiesterase of at least 800 units per gram of solid.

5'-phosphodiesterase activity units are determined as follows: The substrate is a 10 mM solution of bis-(p-nitrophenyl) phosphate (NPP). A solution of 0.1 ml of the enzyme diluted to a concentration of 0.2 to 2.0 units/ml and 0.1 ml of tris/acetate buffer, pH 6.0, and 0.7 ml of water is incubated at 60°C for 1 minute. Then 0.1 ml of NPP solution is added. The reaction is stopped after 10 minutes by the addition of 0.3 ml of sodium carbonate solution. The absorbance at 420 nm is determined. A blank is run in which no enzyme is added to the solutions. The absorbance of the blank is subtracted from the absorbance of the enzyme solution. A phosphodiesterase unit is defined as the amount of enzyme that will produce an absorbance of 1.0 in 1 minute at 60°C, pH 6.0.

5'-nucleotidase activity is determined as follows: The reagent is 5'-guanosine monophosphate. A solution of 0.05 ml of enzyme solution (diluted 1 to 10 in water), 0.05 ml tris/acetate buffer, pH 6.0, and 0.35 ml water is incubated at 60°C for 1 minute. Then 0.05 ml of 100 mM 5'-guanosine monophosphate is added. After 10 minutes, 0.5 ml of 12% trichloroacetic acid is added. After an additional 10 minutes, the amount of inorganic phosphate is determined using the microcolorimetric method of Taussay and Shorr, J. Biol. Chem., 202, 675-685 (1953). A blank is run to determine the amount of inorganic phosphate present in the enzyme solution. A 5'-nucleotidase unit is defined as the amount of enzyme that will release 1 »mole of phosphate per minute at 60°C, pH 6.0.

The 5'-phosphodiesterase enzyme preparation obtained by the process of this invention is an excellent catalyst for the preparation of 5'-nucleotides from RNA. The enzyme preparation is also useful in applications where it is desirable to hydrolyze or remove nucleic acids. For example, it may be used to improve the filterability of a microbial broth. In addition, the enzyme preparation of this invention may be used to treat microbial broths before they are passed over ion-exchange resins. Such treatment greatly reduces the tendency of the microbial broths to foul the resins.

The following examples serve to illustrate the present invention. All parts and percentages are by weight unless otherwise specified.

### EXAMPLE 1

Barley malt sprouts were ground and passed through a screen having a sieve opening of 1.65 mm. To a mixture of 1 part of the ground sprouts and 10 parts by weight of water was added 4.8 g of sodium benzoate and 0.9 g of methyl p-hydroxybenzoate per liter of slurry. Sufficient citric acid solution was added to adjust the pH to 4.6 ± 0.1. The slurry was agitated for 30 minutes. Then the larger solid particles were removed by passing the slurry through a paddle screen apparatus, ICM No. 77, having a screen opening of about 50 microns, manufactured by the Indiana Canning Machine Company. The effluent from the paddle screen, which contained finely-divided solids in suspension, was clarified by passing through a rotary vacuum filter coated with a diatomaceous earth filter aid (CELATOM FW40, available from Eagle-Picher Industries, Inc., Cincinnati, Ohio) with spray water washing. The clarified aqueous enzyme extract was concentrated by means of an ultrafiltration unit having a membrane which retains material having a molecular weight of about 5000 daltons. The solution was concentrated until the enzyme concentration was approximately 30 times that of the clarified aqueous enzyme extract. The concentrate from the ultrafiltration unit was then heated at 63° ± 1°C for 15 minutes by passing it through a heated holding coil. It was then cooled quickly to about 21°C. From 6193 kg of barley malt sprouts there was obtained 802.5 kg of extract containing 120 units/g of 5'-phosphodiesterase enzyme. The ratio of 5'-phosphodiesterase units to 5'-nucleotidase units in this extract was 52:1.

### EXAMPLE 2

The general procedure of Example 1 was repeated except that the first separation step was carried out by means of an Eimco belt press, available from the Process Equipment Company, Palatine, Illinois. The concentrated enzyme preparation contained 73 units/g of 5'-phosphodiesterase enzyme and 1.1 units/g of 5'-nucleotidase units.

A 15.89 kg portion of the enzyme concentrate was freeze-dried using a commercial lyophilizer. The yield of light-brown solid containing 855 units/g of 5'-phosphodiesterase enzyme was 1.21 kg. The ratio of 5'-phosphodiesterase units to 5'-nucleotidase units in this product was about 50:1. The product is stable when stored at room temperature.

## Claims

1. A process for extracting an enzyme preparation from barley malt sprouts which comprises
mixing ground barley malt sprouts with water to give a first aqueous slurry of ground barley malt sprouts;
separating the larger particles of ground barley malt sprouts from said first aqueous slurry in a first separation step to give a second aqueous slurry containing finely-divided barley malt sprouts;
then separating the finely-divided barley malt sprouts from said second aqueous slurry in a second separation step to give a clarified aqueous enzyme extract having less than about 1% by weight of water-insoluble solids;
concentrating the clarified aqueous enzyme extract by ultrafiltration to give a concentrated enzyme extract; and
heating the concentrated enzyme extract at a pH between about 4.3 and about 5.4 at a temperature of between about 60°C and about 65°C for from about 15 to about 60 minutes to give an enzyme preparation having a ratio of 5'-phosphodiesterase units to 5'-nucleotidase units of at least 7:1.

2. The process of claim 1 wherein the barley malt sprouts are ground to pass through a screen having a sieve opening of 1.65 mm.

3. The process of claim 1 wherein the ground barley malt sprouts are mixed with at least 8 parts by weight of water per 1 part by weight of barley malt sprouts.

4. The process of claim 1 wherein the ground barley malt sprouts are mixed with water at a pH between about 4.0 and about 5.0 and at a temperature between about 12°C and about 20°C.

5. The process of claim 1 wherein a preservative is added to the water mixed with the ground barley malt sprouts.

6. The process of claim 5 wherein the preservative is selected from sodium benzoate, methyl p-hydroxybenzoate, and mixtures thereof.

7. The process of claim 1 wherein the first separation step is carried out by means of a paddle screen.

8. The process of claim 1 wherein the second separation step is carried out by means of a rotary precoat filter.

9. The process of claim 1 wherein the ultrafiltration is carried out by means of ultrafiltration equipment having a membrane which retains material having a molecular weight greater than about 5000 daltons.

10. The process of claim 1 wherein the concentrated enzyme extract is heated in a continuous process by passing through a heat exchanger and then cooled rapidly.

11. The process of claim 10 wherein the heated enzyme extract is cooled to a temperature below about 25°C.

12. The process of claim 1 wherein the enzyme preparation has a ratio of 5'-phosphodiesterase units to 5'-nucleotidase units of at least 15:1.

13. An enzyme preparation extracted from barley malt sprouts which comprises 5'-phosphodieserase and 5'-nucleotidase wherein the ratio of 5'-phosphodiesterase units to 5'-nucleotidase units is at least 7:1, which is an aqueous extract and further comprises a preservative.

14. The enzyme preparation of claim 13 wherein the concentration of 5'-phosphodiesterase is at least 100 units per gram of extract.

15. The enzyme preparation of claim 13 or 14 wherein the preservative is selected from the group: sodium benzoate, methyl p-hydroxybenzoate, and mixtures thereof.

16. An enzyme preparation extracted from barley malt sprouts which comprises 5'-phosphodieserase and 5'-nucleotidase wherein the ratio of 5'-phosphodiesterase units to 5'-nucleotidase units is at least 7:1, and wherein the enzyme preparation is a freeze-dried solid.

17. The enzyme preparation of claim 16 wherein the concentration of 5'-phosphodiesterase is at least 800 units per gram of solid.

## Patentansprüche

1. Ein Verfahren zur Extraktion eines Enzympräparats aus Gerstenmalzkeimen, welches umfasst
Mischen von gemahlenen Gerstenmalzkeimen mit Wasser, um eine erste wässrige Aufschlämmung aus gemahlenen Gerstenmalzkeimen zu erhalten;
Abtrennen der grösseren Partikel der gemahlenen Gerstenmalzkeime von genannter erster Aufschlämmumg in einem ersten Trennungsschritt, um eine zweite wässrige Aufschlämmung zu ergeben, die fein getrennte Gerstenmalzkeime enthält;
dann Trennen der fein getrennten Gerstenmalzkeime von genannter zweiten wässrigen Aufschlämmung in einem zweiten Trennungsschritt, um einen geklärten wässrigen Enzymextrakt zu ergeben, der weniger als ungefähr 1 Gewichtsprozent nicht wasserlösliche Feststoffe enthält;
Konzentrieren der gereinigten wässrigen Enzymlösung durch Ultrafiltration, um einen konzentrierten Enzymextrakt zu ergeben; und
Erhitzen des konzentrierten Enzymextrakts bei einem pH zwischen ungefähr 4,3 und ungefähr 5,4 bei einer Temperatur zwischen ungefähr 60°C und ungefähr 65°C für ungefähr 15 bis ungefähr 60 Minuten, um ein Enzympräparat zu ergeben, das ein Verhältnis von 5'-Phosphordiesteraseeinheiten zu 5'Nukleotidaseeinheiten von mindestens 7:1 aufweist.

2. Das Verfahren gemäss Anspruch 1, worin die Gerstenmalzkeime gemahlen werden, um durch ein Sieb mit einer Sieböffnung von 1,65 mm zu fallen.

3. Das Verfahren gemäss Anspruch 1, worin die gemahlenen Gerstenmalzkeime mit mindestens 8 Gewichtsteilen Wasser pro 1 Gewichtsteil Gerstenmalzkeime gemischt werden.

4. Das Verfahren gemäss Anspruch 1, worin die gemahlenen Gerstenmalzkeime bei einem pH zwischen ungefähr 4,0 und ungefähr 5,0 und bei einer Temperatur zwischen 12°C und ungefähr 20°C mit Wasser gemischt werden.

5. Das Verfahren gemäss Anspruch 1, worin ein Konservierungsmittel zu den mit Wasser gemischten gemahlenen Gerstenmalzkeimen gegeben wird.

6. Das Verfahren gemäss Anspruch 5, worin das Konservierungsmittel aus Natriumbenzoesäureester, Methyl-p-hydroxybenzoesäureester und Mischungen davon gewählt wird.

7. Das Verfahren gemäss Anspruch 1, worin die erste Trennung mittels eines Schaufelsiebs durchgeführt wird.

8. Das Verfahren gemäss Anspruch 1, worin die zweite Trennung mittels eines rotierenden Anschwemmfilters durchgeführt wird.

9. Das Verfahren gemäss Anspruch 1, worin die Ultrafiltration mittels einer Ultrafiltrationsapparatur durchgeführt wird, die eine Membran besitzt, die Material mit einem Molekulargewicht grösser als ungefähr 5000 Dalton zurückhält.

10. Das Verfahren gemäss Anspruch 1, worin der konzentrierte Enzymextrakt in einem kontinuierlichen Verfahren durch Passieren eines Hitzeaustauschers erhitzt und dann schnell abgekühlt wird.

11. Das Verfahren gemäss Anspruch 10, worin der erhitzte Enzymextrakt auf eine Temperatur unter 25°C gekühlt wird.

12. Das Verfahren gemäss Anspruch 1, worin das Enzympräparat ein Verhältnis von 5'-Phosphodiesteraseeinheiten zu 5'-Nukleotidaseeinheiten von mindestens 15:1 besitzt.

13. Ein aus Gerstenmalzkeimen extrahiertes Enzympräparat, das 5'Phosphodiesterase und 5'-Nukleotidase umfasst, worin das Verhältnis von 5'-Phosphodiesteraseeinheiten zu 5'-Nukleotidaseeinheiten mindestens 7:1 beträgt, welches ein wässriger Extrakt ist und weiterhin ein Konservierungsmittel umfasst.

14. Das Enzympräparat gemäss Anspruch 13, worin die Konzentration von 5'-Phosphodiesterase mindestens 100 Einheiten pro Gramm Extrakt beträgt.

15. Das Enzympräparat gemäss Anspruch 13 oder 14, worin das Konservierungsmittel aus der Gruppe: Natriumbenzoesäureester, Methyl-p-hydroxybenzoesäureester und Mischungen davon gewählt wird.

16. Ein aus Gerstenmalzkeimen extrahiertes Enzympräparat, das 5'-Phosphodiesterase und 5'-Nukleotidase umfasst, worin das Verhältnis von 5'-Phosphodiesteraseeinheiten zu 5'-Nukleotidaseeinheiten mindestens 7:1 beträgt und worin das Enzympräparat ein gefriergetrockneter Feststoff ist.

17. Das Enzympräparat gemäss Anspruch 16, worin die Konzentration von 5'-Phosphodiesterase mindestens 800 Einheiten pro Gramm Feststoff beträgt.

## Revendications

1. Procédé d'extraction d'une préparation enzymatique de pousses de malt d'orge, qui comprend les étapes consistant
à mélanger des pousses de malt d'orge broyées avec de l'eau pour obtenir une première suspension aqueuse de pousses de malt d'orge broyées ;
à séparer les particules plus volumineuses de pousses de malt d'orge broyées de ladite première suspension aqueuse dans une première étape de séparation, ce qui donne une seconde suspension aqueuse contenant des pousses de malt d'orge finement divisées ;
puis à séparer les pousses de malt d'orge finement divisées de ladite seconde suspension aqueuse dans une seconde étape de séparation, ce qui donne un extrait enzymatique aqueux clarifié renfermant moins d'environ 1 % en poids de matières solides insolubles dans l'eau ;
à concentrer l'extrait enzymatique aqueux clarifié par ultrafiltration, ce qui donne un extrait enzymatique concentré ; et
à chauffer l'extrait enzymatique concentré à un pH d'environ 4,3 à environ 5,4 à une température d'environ 60°C à environ 65°C pendant un temps d'environ 15 à environ 60 minutes, ce qui donne une préparation enzymatique ayant un rapport des unités de 5'-phosphodiestérase aux unités de 5'-nucléotidase d'au moins 7:1.

2. Procédé suivant la revendication 1, dans lequel les pousses de malt d'orge sont broyées de manière à passer à travers un tamis ayant une ouverture des mailles de 1,65 mm.

3. Procédé suivant la revendication 1, dans lequel les pousses de malt d'orge broyées sont mélangées à au moins 8 parties en poids d'eau pour 1 partie en poids de pousses de malt d'orge.

4. Procédé suivant la revendication 1, dans lequel les pousses de malt d'orge broyées sont mélangées à de l'eau à un pH d'environ 4,0 à environ 5,0 et à une température d'environ 12°C à environ 20°C.

5. Procédé suivant la revendication 1, dans lequel un conservateur est ajouté à l'eau mélangée aux pousses de malt d'orge broyées.

6. Procédé suivant la revendication 5, dans lequel le conservateur est choisi entre le benzoate de sodium, le p-hydroxybenzoate de méthyle et leurs mélanges.

7. Procédé suivant la revendication 1, dans lequel la première étape de séparation est mise en oeuvre au moyen d'un crible à pales.

8. Procédé suivant la revendication 1, dans lequel la seconde étape de séparation est mise en oeuvre au moyen d'un filtre rotatif à couches.

9. Procédé suivant la revendication 1, dans lequel l'ultrafiltration est effectuée au moyen d'un appareil d'ultrafiltration possédant une membrane qui retient les substances ayant un poids moléculaire supérieur à environ 5000 daltons.

10. Procédé suivant la revendication 1, dans lequel l'extrait enzymatique concentré est chauffé dans un processus continu par passage à travers un échangeur de chaleur, puis est refroidi rapidement.

11. Procédé suivant la revendication 10, dans lequel l'extrait enzymatique chauffé est refroidi à une température inférieure à environ 25°C.

12. Procédé suivant la revendication 1, dans lequel la préparation enzymatique a un rapport des unités de 5'-phosphodiestérase aux unités de 5'-nucléotidase d'au moins 15:1.

13. Préparation enzymatique extraite de pousses de malt d'orge, qui comprend de la 5'-phosphodiestérase et de la 5'-nucléotidase, dans laquelle le rapport des unités de 5'-phosphodiestérase aux unités de 5'-nucléotidase est au moins égal à 7:1, qui est un extrait aqueux et qui comprend en outre un conservateur.

14. Préparation enzymatique suivant la revendication 13, dans laquelle la concentration de 5'-phosphodiestérase est au moins égale à 100 unités par gramme d'extrait.

15. Préparation enzymatique suivant la revendication 13 ou 14, dans laquelle le conservateur est choisi dans le groupe consistant en benzoate de sodium, p-hydroxybenzoate de méthyle et leurs mélanges.

16. Préparation enzymatique extraite de pousses de malt d'orge, qui comprend de la 5'-phosphodiestérase et de la 5'-nucléotidase, dans laquelle le rapport des unités de 5'-phosphodiestérase aux unités de 5'-nucléotidase est au moins égal à 7:1, ladite préparation enzymatique étant sous forme d'une substance solide lyophilisée.

17. Préparation enzymatique suivant la revendication 16, dans laquelle la concentration de 5'-phosphodiestérase est au moins égale à 800 unités par gramme de substance solide.
